# EUROPEAN PATENT APPLICATION

(11) **EP 2 873 367 A1**
(43) Date of publication of application: **20.05.2015**
(21) Application number: 14193295.4
(22) Date of filing: 14.11.2014
(51) Int. Cl.: A61B 5/11, A61B 5/22, A61B 5/00, A61B 5/024

(54) **Recognition device of foot action and recognition method of foot action**

(30) Priority: 15.11.2013 TW 102141792; 11.11.2014 TW 103139097
(71) Applicant: Leu, Chun Yi, Taipei 114 (TW)
(72) Inventor: Leu, Chun Yi, Taipei 114 (TW)
(74) Representative: Epping - Hermann - Fischer

(57) **Abstract**

A recognition device of a foot action includes a sensing module, a processing module, and a display module. The sensing module senses the foot action to generate at least one sensing signal corresponding to the foot action, and a form of the sensing signal is a waveform. The processing module is electrically connected the sensing module. The processing module recognizes an action mode corresponding to the foot action according to the waveform and converts the waveform into input data according to the action mode. The display module is electrically connected the processing module and displays the input data. Furthermore, a recognition method of the foot action is also disclosed herein.

## Description

### CROSS-REFERENCE TO RELATED APPLICATION(S)

This application claims priority to Taiwan Patent Application No. 102141792 filed on November 15, 2013 and Taiwan Patent Application No. 103139097 filed on November 11, 2014, which is herein incorporated by reference.

### BACKGROUND

### Field of Invention

The present disclosure relates to a recognition device. More particularly, the disclosure relates to a recognition device of a foot action and a recognition method of the foot action.

### Description of Related Art

Obesity has recently become the leading threaten of health worldwide, mainly because of changes in lifestyle and diet nowadays. With increasing prevalence of obesity, which induces various cardiovascular diseases, type 2 diabetes, and even certain types of cancer in population, authorities has started emphasizing the importance of weight control. According to medical researches and nutritionists, adequate exercise is considered as an appropriate way of weight control instead of dieting. However, the ideal quantity of exercise varies depending on factors such as gender, age, smoking, gene, and body mass index (BMI).

To quantify exercise intensity, people usually use recognition devices while exercising to gather numeric information, and further optimize exercise plans by that information. One of the most common recognition devices is pedometer. A pedometer was positioned about a user's hip and counts each step the user takes by detecting the motion. An advanced pedometer also monitors distance, velocity, heart rate, and calorie burned of a user. Another example of a sensing device is sport watch. Some sport watches are capable of monitoring distance, duration, heart rate, and calorie burned of an exercise; they are also capable of operating under different situations including walking, running, cycling, or marathon.

Now, there is a cadence meter to measure the frequency of stepping the footrest on the bicycle. It is set a magnet on the footrest or the wheel of the bicycle, then the magnet triggers the cadence meter to measure the rotational speed of the wheel. However, the cadence meter is set hardly, and it merely measures the frequency of stepping the footrest on the bicycle, so that it cannot be used when the user is running or walking.

Conventional pedometer merely measure for running and walking but not satisfy the users for every kind of sports. A sport watch is compatible with several different exercises, but the interaction is restricted between the user and the sport watch. Therefore, it is one of the important subjects and the objective needs to be improved in the related areas to recognize the action modes of the different foot action, measure the relative value of the foot action, and display the action mode and the relative value of the foot action synchronously.

### SUMMARY

A recognition device of a foot action and a recognition method of the foot action are provided to resolve the problems of the prior art.

According to an embodiment of the present invention, the recognition device of the foot action includes a sensing module, a processing module, and a display module. The sensing module is configured to sense the foot action to generate at least one sensing signal corresponding to the foot action, and a form of the sensing signal is a waveform. The processing module is electrically connected the sensing module and configured to recognize an action mode corresponding to the foot action according to the waveform, and the action mode corresponding to the foot action according to the waveform is converted into output data. The display module is electrically connected the processing module and configured to display the output data.

In an embodiment, the sensing module is selected from a gyroscope, an accelerometer, an electron compass, a heart rate sensor, a humidity sensor, and a global positioning system (GPS) sensor.

In an embodiment, the recognition device further includes a storage module that is electrically connected the processing module and configured to store output data.

In an embodiment, the recognition device further includes a transmission module that is electrically connected the processing module and configured to transmit the output data to a receiving device through a network, and the transmission module is selected from a wide area network (WAN) module, a bluetooth module, a ANT+ module, and a local area network (LAN) module.

In an embodiment, the receiving device further includes a receiving module, a microprocessor, and a display unit. The receiving module is electrically connected the microprocessor and configured to receive the output data transmitted by the transmission module through the network. The microprocessor is electrically connected the receiving module and configured to convert the output data received by the receiving module into display data. The display unit is electrically connected the microprocessor and configured to display the display data.

In an embodiment, the action mode is selected from a working mode, a stair climbing mode, a bicycle mode, a swimming mode, and a sleeping mode, and the stair climbing mode includes a stair ascending mode and a stair descending mode. The swimming mode includes a freestyle mode and a breaststroke mode.

In an embodiment, the processing module recognizes the action mode as one of the working mode, the stair climbing mode, and the bicycle mode according to the waveform when a cycle of the waveform comprises only a wave crest that the normal vector value of the wave crest exceeds a determine value. The processing module recognizes the action mode as the swimming mode according to the waveform when a cycle of the waveform comprises more two wave crests that the normal vector value of the wave crest exceeds the determine value.

In an embodiment, when the action mode is one of the working mode, the stair climbing mode, and the bicycle mode, the processing module recognizes the action mode as the bicycle mode if the cycle of the waveform comprises a similar sinusoidal wave.

In an embodiment, the processing module recognizes the action mode as one of the working mode, the stair ascending mode of the stair climbing mode, and the stair descending mode of the stair climbing mode according to a grounding interval of the waveform.

In an embodiment, the processing module recognizes the action mode as the stair ascending mode when the maximum value of the grounding interval of the waveform is larger than the zero normal vector value, the processing module recognizes the action mode as the stair descending mode when the maximum value of the grounding interval of the waveform is smaller than the zero normal vector value, and the processing module recognizes the action mode as the walking mode when the maximum value of the grounding interval of the waveform is equal to the zero normal vector value.

In an embodiment, when the action mode is the swimming mode, the processing module recognizes the action mode as the freestyle mode of the swimming mode if the cycle of the waveform comprises more one sinusoidal wave.

According to an embodiment of the present invention, a recognition method of a foot action includes: sensing the foot action to generate at least one sensing signal corresponding to the foot action, and a form of the sensing signal is a waveform; recognizing an action mode corresponding to the foot action according to the waveform, and converting the waveform into output data corresponding to the action mode; and displaying the output data.

In an embodiment, the recognition method further includes storing the output data.

In an embodiment, the recognition method further includes transmitting the output data to a receiving device through a network.

In an embodiment, wherein when the receiving device through the network receives the output data, the receiving device converts the output data received into display data and displays the display data in the receiving device.

In an embodiment, the action mode is one selected from a working mode, a stair climbing mode, and a bicycle mode, a swimming mode, and a sleeping mode, and the stair climbing mode is one selected from a stair ascending mode and a stair descending mode, and the swimming mode is one selected from a freestyle mode and a breaststroke mode.

In an embodiment, the recognition method further includes: recognizing the action mode as one of the working mode, the stair climbing mode, and the bicycle mode according to the waveform when a cycle of the waveform comprises only a wave crest that the normal vector value of the wave crest exceeds a determine value; and recognizing the action mode as the swimming mode according to the waveform when a cycle of the waveform comprises more two wave crests that the normal vector value of the wave crest exceeds the determine value.

In an embodiment, the recognition method further includes recognizing the action mode as the bicycle mode if the cycle of the waveform comprises a similar sinusoidal wave when the action mode is one of the working mode, the stair climbing mode, and the bicycle mode.

In an embodiment, the recognition method further includes recognizing the action mode as one of the working mode, the stair ascending mode of the stair climbing mode, and the stair descending mode of the stair climbing mode according to a grounding interval of the waveform.

In an embodiment, the recognition method further includes recognizing the action mode as the stair ascending mode when the maximum value of the grounding interval of the waveform is larger than the zero normal vector value; recognizing the action mode as the stair descending mode when the maximum value of the grounding interval of the waveform is smaller than the zero normal vector value; and recognizing the action mode as the walking mode when the maximum value of the grounding interval of the waveform is equal to the zero normal vector value.

In an embodiment, the recognition method further includes recognizing the action mode as the freestyle mode of the swimming mode if the cycle of the waveform comprises more one sinusoidal wave.

In summary, the present invention has significant advantages and higher performance than current technology. With the present invention, we have significant improvement in the recognizing techniques for the foot action, and it is worthwhile to be widely used and implemented in industry. Advantages of the present invention are to recognize the action modes of the different foot action, measure the relative value of the foot action, and display the action mode and the relative value of the foot action synchronously.

It is to be understood that both the foregoing general description and the following detailed description are by examples, and are intended to provide further explanation of the invention as claimed.

### BRIEF DESCRIPTION OF THE DRAWINGS

The invention can be more fully understood by reading the following detailed description of the embodiment, with reference made to the accompanying drawings as follows:
Fig. 1 is a block diagram of a recognition device of a foot action according to an embodiment of the present invention;
Fig. 2A is a waveform diagram of an action mode as a working mode according to an embodiment of the present invention;
Fig. 2B is a waveform diagram of an action mode as a stair climbing mode according to an embodiment of the present invention;
Fig. 2C is a waveform diagram of an action mode as a bicycle mode according to an embodiment of the present invention;
Fig. 3A is a waveform diagram of an action mode as a freestyle mode of a swimming mode according to an embodiment of the present invention;
Fig. 3B is a waveform diagram of an action mode as a breaststroke mode of a swimming mode according to an embodiment of the present invention;
Fig. 4 is a schematic diagram of manually switching action modes of a recognition device according to an embodiment of the present invention; and
Fig. 5 is a flow chart of a recognition method of a foot action according to an embodiment of the present invention.

### DETAILED DESCRIPTION

When the user moves or sports, the foots of the user usually repeat the action regularly. However, the hands of the user may keep the same pose or swing by manner. For instance, when the user rides the bicycle, the hands of the user hold the grips of the bicycle, and the foots of the user step on the footrests of the bicycle regularly. For the hand action and the foot action, the variation of the hand is more obvious than the variation of the foot. When the user walks, the foots of the user take steps regularly, but the hands of the user may keep the same pose or swing by manner.

A recognition device of the foot action disclosed herein is to sense the foot action of the user to recognize the action that the user is doing, such as walking, climbing, riding the bicycle, swimming, dancing, sleeping. The action that the user is doing may be recognized according to the different regular action of the foot action, and the data relates to the action that the user is doing further may be measured, such as the amount of walking step, the speed of walking, the mileage of walking, the speed of stair climbing, the rotational speed of the bicycle, the way of swimming, the frequency of the heart.

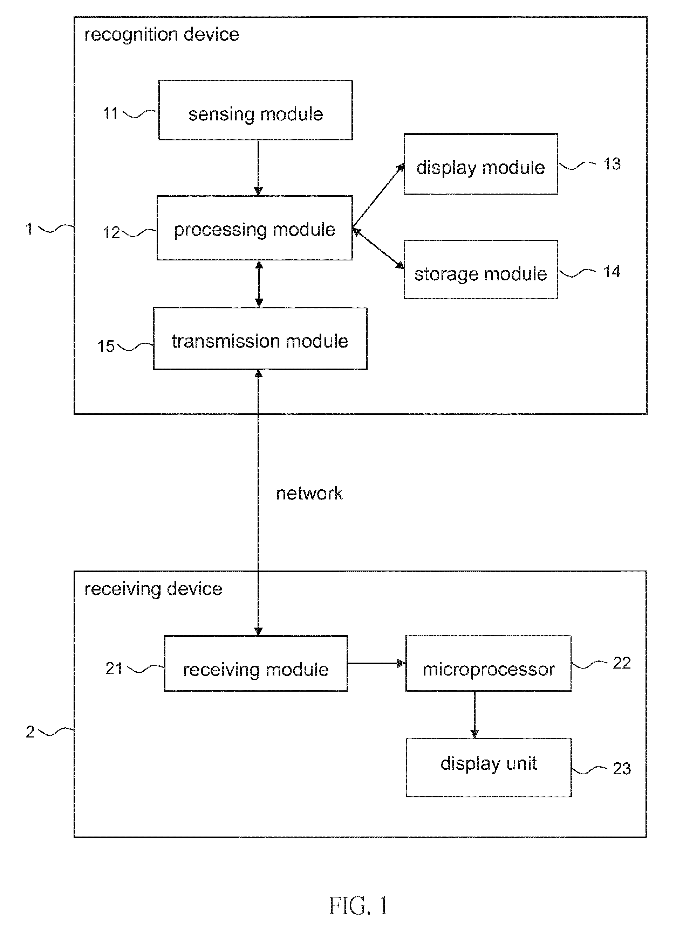

Fig. 1 is a block diagram of a recognition device 1 of a foot action according to an embodiment of the present invention. As shown in Fig. 1, according to an embodiment of the present invention, the recognition device 1 of the foot action includes a sensing module 11, a processing module 12, and a display module 13. The sensing module 11 is configured to sense the foot action generate at least one sensing signal corresponding to the foot action, and a form of the sensing signal is a waveform. The processing module 12 is electrically connected the sensing module 11. The processing module 12 is configured to recognize an action mode corresponding to the foot action according to the waveform and convert the waveform into the output data corresponding to the action mode. The display module 13 is electrically connected the processing module 12 and configured to display the output data that the processing module 12 converts the waveform corresponding to the action mode.

In practice, the recognition device 1 may be a device fastened on a foot of the user (e.g., below the lower leg). For example, the recognition device 1 may be a foot ring, also be a device additionally fastened on a foot ring that the user owns, further be a device fastened on a shoe that the user owns. It should be understood that none of the above examples is more preferable than any of the other examples, nor are they intended to limit the scope of the invention. Those skilled in the art may specifically implement the recognition device 1 with flexibility according to the requirements then.

When the user processes the different actions such as walking, climbing, stair climbing, riding bicycle, swimming, dancing, sleeping, the foots may generate the repeat action relates to the different actions. When the user walks, stair ascends or stair descends normally, the foots will take steps by a fixed frequency within a time period (e.g., 3 secs), and the distance between two steps is nearly same. When the user rides the bicycle normally, the foots will step on the footrest of the bicycle by a fixed frequency within a time period (e.g., 1 sec), and the effort per stepping on the footrest is nearly same. When the user swims normally, no matter which swim way (e.g., freestyle, breaststroke, butterfly), the foots will swing to move the body by a fixed frequency within a time period (e.g., 1 sec), and the effort per swinging is nearly same. When the user sleeps, the foots will stop moving. The recognition device 1 fastened on the foot of the user may sense the foot action to generate a sensing signal of the foot action. Because the repeat action of the foot is processed by the different frequency according to the state of the action when the user processes the different action, the waveform corresponding to the sensing signal may have the different form, period, and amplitude.

After the sensing module 11 senses the foot action of the user to generate the waveform corresponding to the sensing signal, the processing module 12 recognizes the action mode of the foot action according to the form, period, and amplitude of the waveform. For example, the action mode corresponding to the foot action is a walking mode when the foot action of the user is walking. The action mode corresponding to the foot action is a climbing mode or a stair climbing mode when the foot action of the user is climbing or stair climbing. The action mode corresponding to the foot action is a bicycle mode, a swimming mode, a dancing mode, or a sleeping mode respectively when the foot action of the user is riding bicycle, swimming, dancing, or sleeping. Any foot action of the user has the corresponding action mode, and none of the above examples is not limited the scope of the invention.

In an embodiment, the sensing module 11 of the recognition device 1 is selected from a gyroscope, an accelerometer, an electron compass, a heart rate sensor, a humidity sensor, a global positioning system (GPS) sensor or a sensing device that senses the foot action to generate the waveform.

When the user processes the different action, the foot action corresponding to the different action may repeat along a different displacement path. The waveform corresponding to the sensing signal relates to the displacement path. The processing module 12 recognizes action mode corresponding to the foot action according to the form of the waveform.

The processing module 12 as mentioned previously may be specifically implemented in software or hardware and/or firmware. For example, if the operating speed and precision are both the first considerations, the processing module 12 may be mainly implemented in hardware and/or firmware. If design flexibility is the first consideration, the processing module 12 are mainly implemented in software. Alternatively, the processing module 12 operate with the collaboration of software, hardware, and firmware. It should be understood that none of the above examples is more preferable than any of the other examples, nor are they intended to limit the scope of the invention. Those skilled in the art may specifically implement the processing module 12 with flexibility according to the requirements then.

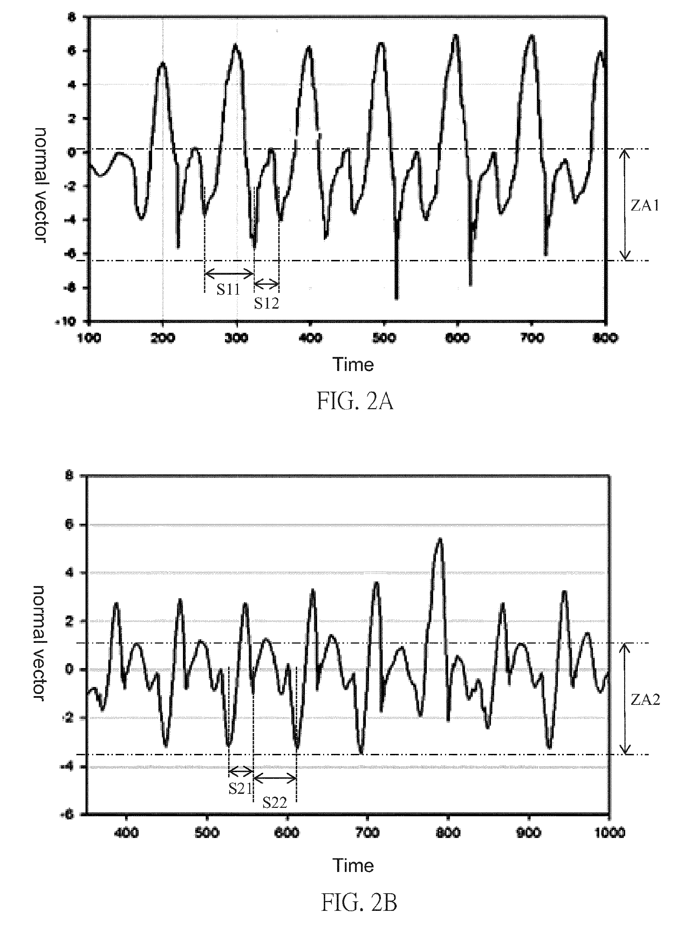

Fig. 2A is a waveform diagram of an action mode as the working mode or a running mode according to an embodiment of the present invention. As shown in Fig. 1 and Fig. 2A, when the user walks, the foots of the user take steps by turns and repeat the same pose. The sensing module 11 senses one foot of the user to generate the waveform corresponding to the sensing signal when the user raises the one foot to take s step, and a normal vector value of the waveform may increase by the positive direction and increase to much higher than a zero normal vector value. The sensing module 11 senses the one foot of the user to generate the waveform corresponding to the sensing signal when the user puts down the one foot, and the normal vector value of the waveform may decrease by the negative direction. The waveform during an interval S11 corresponds to the pose that the user raises the one foot then puts down it.

Afterwards, for the normal pose of a human walking or running, the front part of the one foot of the user may not leave the ground but the heel of the one foot of the user may leave the ground during the user raises another foot then puts down the another foot. The sensing module 11 senses the one foot of the user to generate the waveform corresponding to the sensing signal, and the normal vector value of the waveform may increase by the positive direction but just increase to near the zero normal vector value. The waveform during an interval S12 corresponds to the pose of the one foot of the user during the another foot of the user raising then putting down. The continuous waveform during the interval S11 and the interval S12 only includes one wave crest, and the normal vector value of the wave crest is large that a determine value (e.g., 2).

When the action mode is the working mode, the maximum value of a grounding interval ZA1 is near the zero normal vector value because the altitude of the foot touching the ground this time is the nearly same with the altitude of the foot touching the ground last time.

Fig. 2B is a waveform diagram of an action mode as the stair climbing mode according to an embodiment of the present invention. The stair climbing mode includes a stair ascending mode and a stair descending mode. Because the foot actions are the same when the user climbs and stair climbs, the following embodiment is merely descripted according to the stair climbing mode. i.e., the stair ascending mode includes a uphill mode, and the stair descending mode includes a downhill mode. As shown in Fig. 1 and Fig. 2B, The sensing module 11 senses the one foot of the user to generate the waveform corresponding to the sensing signal when the user raises the one foot to take a stair step, and the normal vector value of the waveform may increase by the positive direction and increase to much higher than the zero normal vector value. The sensing module 11 senses the one foot of the user to generate the waveform corresponding to the sensing signal when the user puts down the one foot, and the normal vector value of the waveform may decrease by the negative direction. The waveform during an interval S21 corresponds to the pose that the user raises the one foot to take the stair step then puts down it. The waveform during an interval S22 corresponds to the pose of the one foot of the user during the another foot of the user raising to take the stair step then putting down.

The stair climbing mode may be recognized as the stair ascending mode or the stair descending mode by the processing module 12 according to a grounding interval ZA2 of the waveform. When the action mode is the stair ascending mode of the stair climbing mode, the maximum value of a grounding interval ZA2 is higher than the zero normal vector value because the altitude of the foot touching the ground this time is higher than the altitude of the foot touching the ground last time. On the other hand, When the action mode is the stair descending mode of the stair climbing mode, the maximum value of a grounding interval ZA2 is lower than the zero normal vector value because the altitude of the foot touching the ground this time is lower than the altitude of the foot touching the ground last time. The continuous waveform during the interval S21 and the interval S22 only includes one wave crest, and the normal vector value of the wave crest is large that the determine value (e.g., 2).

From the foregoing, regardless of the user walking or stair climbing, the foots of the user take steps by turns and repeat the same pose. For the normal poses of a human walking and stair climbing, the ways of taking steps are the same. However, the altitude of the foot touching the ground every time is the same with the altitude last time when the user walks, but the altitude of the foot touching the ground every time is different from the altitude last time when the user stair ascending or stair descending. Therefore, as shown in Fig. 1, 2A, 2B, when the waveform corresponding to the sensing signal is like as the waveform during the interval S11 or the interval S21 that the normal vector value of the waveform may increase by the positive direction and increase to much higher than a zero normal vector value, the action mode may be recognized as the walking mode or the stair climbing mode by the processing module 12 according to the waveform. Afterwards, the action mode may be recognized as one of the walking mode, the stair ascending mode and the stair climbing mode by the processing module 12 according to whether the maximum value of the grounding interval ZA2 is larger than, lower than, or equal to the zero normal vector value.

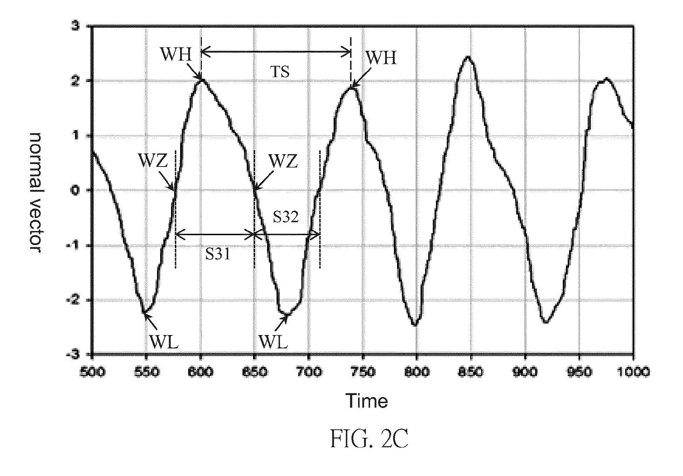

Fig. 2C is a waveform diagram of an action mode as the bicycle mode according to an embodiment of the present invention. As shown in Fig. 2C, when the user rides the bicycle, the form of the waveform shows as a sinusoidal wave because the position of the foot may repeatedly change between the highest position and the lowest position of the footrest. When the position of the one foot locates on the highest position, the position of the another foot locates on the lowest position. When both the one foot and the another foot locate on the same, the position of both foots locates on the middle position between the highest position and the lowest position.

When the one foot of the user steps on the footrest from the highest position to the lowest position, the sensing module 11 senses the one foot of the user to generate the sensing signal shown as the waveform. The normal vector value of the waveform may increase by the positive direction and increase to much higher than a zero normal vector value. The waveform during an interval S31 corresponds to the pose that the one foot of the user steps on the footrest from the highest position to the lowest position. When the one foot of the user steps on the footrest from the highest position to the lowest position, the sensing module 11 senses the one foot of the user to generate the sensing signal shown as the waveform. The normal vector value of the waveform may decrease by the negative direction. The waveform during an interval S32 corresponds to the pose that the one foot of the user steps on the footrest from the lowest position to the highest position. The continuous waveform during the interval S31 and the interval S32 is a similar regular sinusoidal wave obviously. The continuous waveform corresponds that the foot action of the user is a regular circular motion when the user rides the bicycle. And, the continuous waveform during the interval S31 and the interval S32 only includes one wave crest, and the normal vector value of the wave crest is large that the determine value (e.g., 2).

Furthermore, When the position of the foot locates on the highest position, the position of the wave crest position WH locates much higher than the zero normal vector value. When the position of the foot locates on the lowest position, the position of the wave trough position WL locates much lower than the zero normal vector value. When the position of the foot locates on the middle position, the position of the wave middle position WZ locates the zero normal vector value.

It is worth mentioning, when the action mode is the walking mode, the climbing mode, the stair climbing, the bicycle mode, or the swimming mode, the waveforms between the one foot and the another foot differ 180 degrees phase because the both foots of the user take steps by turns and repeat the same pose.

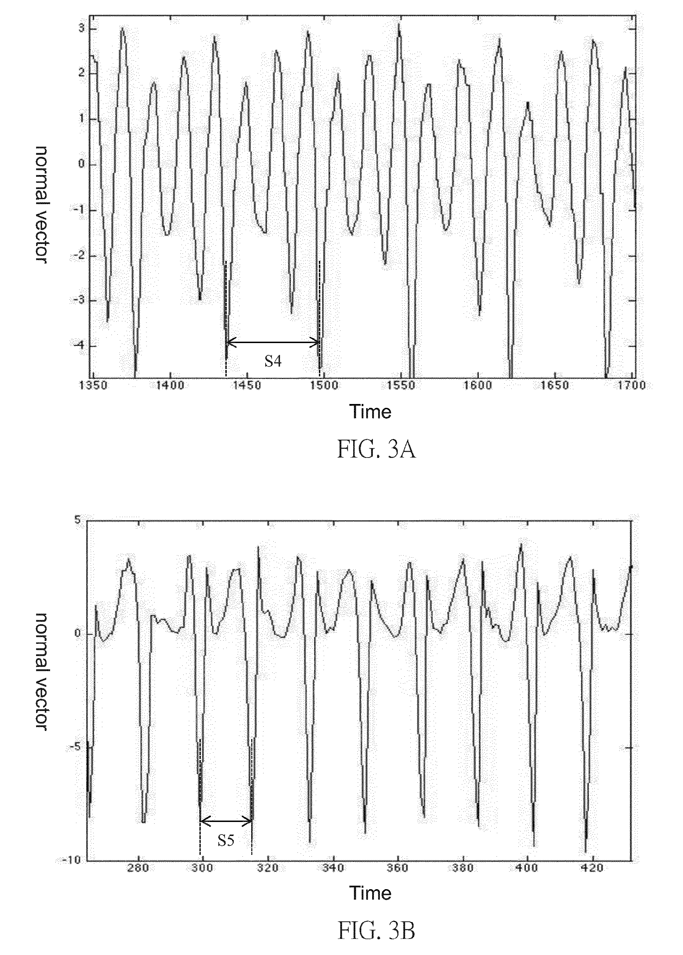

In an embodiment, the swimming mode includes the freestyle mode the breaststroke mode. When the action mode is the swimming mode, the processing module 12 may recognize the action mode as the freestyle mode of the swimming mode or the breaststroke mode according to the form of the waveform so that the swimming way of the user may be known. Fig. 3A is a waveform diagram of an action mode as the freestyle mode of the swimming mode according to an embodiment of the present invention. As shown in Fig. 1 and 3A, when the swimming way of the user is freestyle, the foot action of the user is a circular motion but not the regular circular motion such as riding the bicycle. The sensing module 11 senses the foot action of the user to generate the waveform that is merely similar to the sinusoidal wave and different from the sinusoidal wave shown in Fig. 2C. The waveform during an interval S4 includes more one wave crest, and the normal vector value of the wave crest is large that the determine value (e.g., 2).

Fig. 3B is a waveform diagram of an action mode as the breaststroke mode of a swimming mode according to an embodiment of the present invention. As shown in Fig. 1 and 3B, when the swimming way of the user is breaststroke, the foot action of the user is not the circular motion. The sensing module 11 senses the foot action of the user to generate the waveform, and the waveform is not near the sinusoidal wave that obviously differs from the waveform as shown in Fig. 3A. The waveform during an interval S5 includes more one wave crest, and the normal vector value of the wave crest is large that the determine value (e.g., 2).

As mentioned previously, the processing module 12 as shown in Fig. 1 recognizes the action mode according to the form of the waveform. The processing module 12 recognizes the action mode as one of the walking mode, the stair climbing, and the bicycle mode when one period of the waveform includes only one wave crest that its normal vector value is large that the determine value (e.g., 2). On the other hand, the processing module 12 recognizes the action mode as the swimming mode when one period of the waveform includes more one wave crest that its normal vector value is large that the determine value (e.g., 2).

When the processing module 12 recognizes the action mode as one of the walking mode, the stair climbing, and the bicycle mode, the processing module 12 may recognize the action mode as the bicycle mode if one period of the waveform includes the similar sinusoidal wave. On the other hand, The processing module 12 may recognize the action mode as one of the walking mode and the stair climbing mode if one period of the waveform does not include the similar sinusoidal wave.

When the processing module 12 recognizes the action mode as one of the walking mode and the stair climbing, the processing module 12 recognizes the action mode as one of the walking mode, the stair ascending mode and the stair climbing mode according to whether the maximum value of the grounding interval ZA2 is larger than, lower than, or equal to the zero normal vector value.

Furthermore, when the processing module 12 recognizes the action mode as the swimming mode, the processing module 12 may recognize the action mode as the swimming mode if the waveform in one period includes the similar sinusoidal wave. On the other hand, the processing module 12 may recognize the action mode as the breaststroke mode of the swimming mode if the waveform in one period does not include the similar sinusoidal wave.

Next, as shown in Fig. 1, the processing module 12 converts the waveform into the output data corresponding to the action mode. In an embodiment, the output data may be the amount of walking step, the speed of walking, the mileage of walking, the speed of stair climbing, the rotational speed of the bicycle, the way of swimming, the heart rate, or any data converted from the waveform.

In an embodiment, when the sensing module 11 is the accelerometer, the accelerometer may measure the direction of the gravity according to the sensing signal generated from the accelerometer. In an embodiment, when the sensing module 11 is the gyroscope, the gyroscope may measure the angle of inclination for the foot of the user according to the sensing signal generated from the accelerometer. No matter the sensing module 11 is the accelerometer or the gyroscope, the pose of the user such as standing, lying down, or wrestling may be recognized according to the sensing signal.

In an embodiment, when the sensing module 11 is the humidity sensor, the processing module 12 may senses that whether the recognition device 1 locates in water according to the sensing signal generated from the humidity sensor to recognize whether the user swims.

In an embodiment, when the sensing module 11 is the electron compass, the processing module 12 may recognize the direction that the user faces according to the sensing signal generated from the electron compass and further know the circle amount of the user running back and forth, the circle amount of the user running in a parade ground, the amount of the user swimming back and forth, or determine whether the user processes the indoor sport (e.g., a running machine, a fly wheel).

In an embodiment, when the sensing module 11 is the GPS sensor, the processing module 12 may recognize the location and the sport path of the user according to the sensing signal generated from the GPS sensor and further know the output date corresponding to the relative action mode (e.g., the heart rate, the speed of running, the frequency of the steps, the frequency of the step on, the data of the uphill and the downhill).

In an embodiment, when the sensing module 11 is an infrared heart rate sensor, the infrared heart rate sensor may sense the heart rate. The infrared light radiated from the infrared heart rate sensor may radiate the blood vessel in the skin through the skin then reflect to the infrared heart rate sensor via the boon. The infrared heart rate sensor may convert a pulse corresponding to the blood vessel in the skin into a pulse wave of the current then transmit the pulse wave of the current to the processing module 12. The processing module 12 processes the pulse wave of the current then generate the output data corresponding to the pulse so that the heart rate may be known.

In an embodiment, the sensing module 11 may the frequency of stepping the footrest when the user rides the bicycle. As shown in Fig. 1 and 2C, the sensing module 11 senses the foot action of the user riding the bicycle to generate the sensing signal and transmit the sensing signal to the processing module 12. The processing module 12 may filter the waveform of the sensing signal to remove the noise and recognize the wave crest position WH, the wave trough position WL or the middle position WZ of the waveform. A time difference TS is calculated from the distant two wave crest positions WH, the distant wave trough positions WL or the distant middle positions WZ. the processing module 12 may calculate the frequency of stepping the footrest when the user rides the bicycle after the cumulative period of time.

As shown in Fig. 1, in an embodiment, the recognition device 1 further includes a storage module 14. The storage module 14 is electrically connected the processing module 12 and configured to store the output data corresponding to the action mode converted the waveform by the processing module 12.

In an embodiment, the recognition device 1 further includes the transmission module 15. The transmission module 15 is electrically connected the processing module 12 and configured to transmit the output data to the receiving device 2 through the network (e.g., 3G communication network, 4G communication network, 2.4G communication network, Bluetooth communication network, ANT+ communication network or Wi-Fi communication network).

In an embodiment, the transmission module 15 may be a wide area network (WAN) module (e.g., a 3G communication module, a 4G communication module), a 2.4G communication module, a bluetooth module, a ANT+ module, or a local area network (LAN) module (e.g., Wi-Fi communication network).

In an embodiment, the receiving device 2 includes a receiving module 21, a microprocessor 22, and a display unit 23. The receiving module 21 is configured to receive the output data transmitted from the transmission module 15 through the network (e.g., 3G communication network, 4G communication network, 2.4G communication network, Bluetooth communication network, ANT+ communication network, or Wi-Fi communication network). The microprocessor 22 is electrically connected the receiving module 21 and configured to convert the output data received from the receiving module 21 into the display data. The display unit 23 is electrically connected the microprocessor 22 and configured to display the display data.

In practice, the receiving device 2 may be a computer device (e.g., a desktop computer, a laptop), a portable device (e.g., a smart phone, a tablet computer), or a monitor that includes the microprocessor, and the computer device, the portable device, and the monitor that can connect the network.

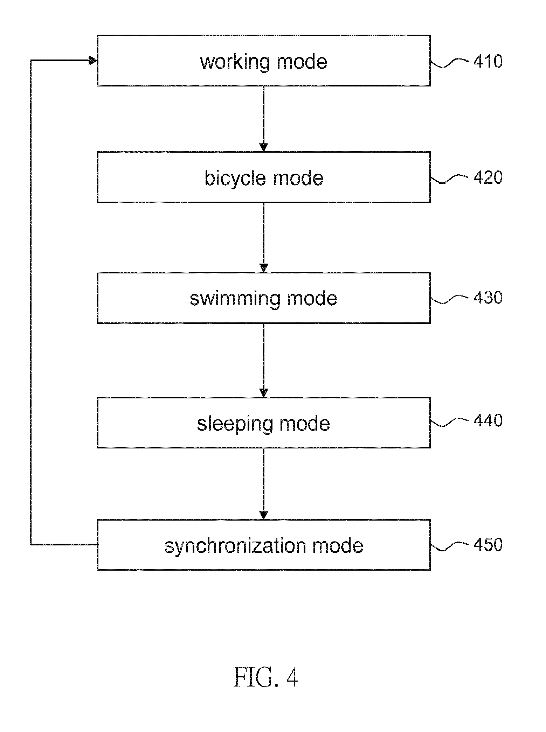

In an embodiment, the user may touch the recognition device 1 manually to switch all kinds of the action modes. Fig. 4 is a schematic diagram of manually switching action modes of a recognition device according to an embodiment of the present invention. As shown in Fig. 1 and 4, the user touches the recognition device 1 manually to sequentially switch the recognition device 1 to a working mode 410, a bicycle mode 420, a swimming mode 430, a sleeping mode 440, a synchronization mode 450.

For example, when the user touches the recognition device 1 manually to switch the recognition device 1 to the working mode 410, the sensing module 11 senses the foot action of the user to generate the sensing signal corresponding to the foot action and transmits the sensing signal to the processing module 12. After the processing module 12 receives the sensing signal, the processing module 12 converts the waveform corresponding to the sensing signal into the output data (e.g., the amount of walking step, the speed of walking, the mileage of walking, or the heart rate) corresponding to the working mode 410 because the recognition device 1 is switched to the working mode 410.

And so on, when the user touches the recognition device 1 manually to switch the recognition device 1 to the bicycle mode 420, the swimming mode 430, or the sleeping mode 440, the processing module 12 converts the waveform corresponding to the sensing signal into the output data of corresponding to the bicycle mode 420, the swimming mode 430, or the sleeping mode 440, and the display module 13 displays the output data.

It is worth noting that as long as the recognition device 1 does not be switched to the sleeping mode 440 manually, the recognition device 1 will enter the sleep state and break down in order to reduce the power consumption of the recognition device 1 if the user has no action within a time interval (e.g., 10 minutes).

When the user touches the recognition device 1 manually to switch the recognition device 1 to the synchronization mode 450, the recognition device 1 may transmit the output data to the receiving device 2 thought the transmission module 15 and the network. In an embodiment, when the receiving device 2 is the computer device, the portable device, or the monitor that includes the microprocessor, the user may switch the recognition device 1 to the synchronization mode 450 so that the output data converted from the recognition device 1 is synchronized to a game program in the receiving device 2, and the user may control the game program in the receiving device 2 through the recognition device 1 fastened on the foot.

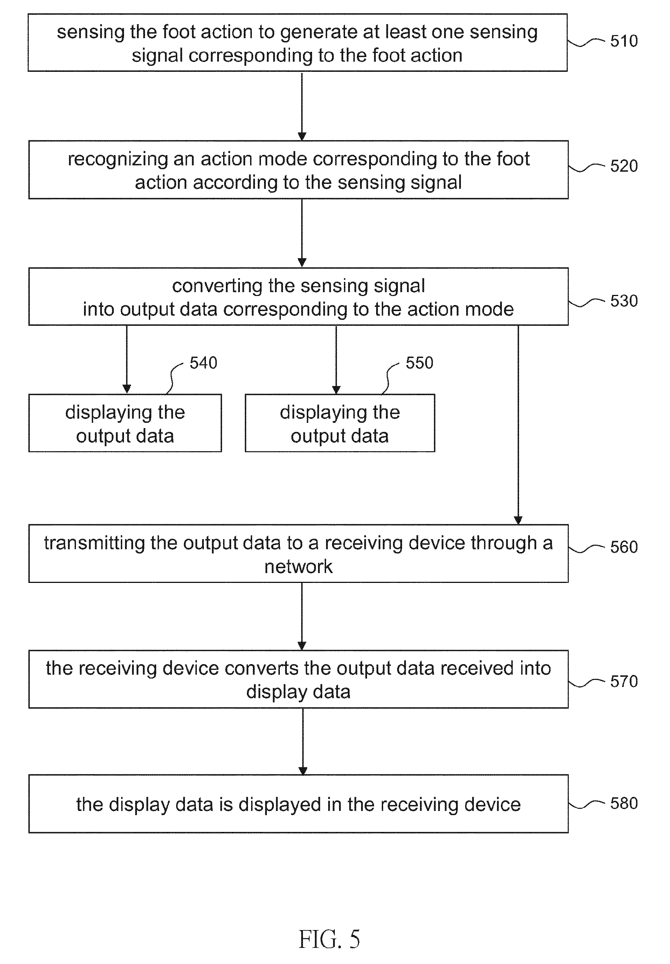

Fig. 5 is a flow chart of a recognition method of a foot action according to an embodiment of the present invention. As shown in Fig. 5, the recognition method of the foot action comprises steps 510 - 580 (it should be understood that the order of the steps mentioned in the present embodiment may be changed based on actual requirement unless otherwise specified, or the steps may even be performed simultaneously or part of the steps may even be performed simultaneously). Since the hardware devices for implementing these steps have been specifically disclosed in the above embodiments, further elaboration is not provided.

In the step 510, the foot action is sensed to generate at least one sensing signal corresponding to the foot action, and a form of the sensing signal is a waveform. In the step 520, an action mode of the foot action (e.g., a walking mode, a climbing mode, a stair climbing mode, a bicycle mode, a swimming mode, a dancing mode, a sleeping mode, or the action mode corresponding to the foot action) is recognized according to the waveform of the sensing signal. In the step 530, the waveform of the sensing signal is converted into output data of the action foot (e.g., the amount of walking step, the speed of walking, the mileage of walking, the speed of stair climbing, the rotational speed of the bicycle, the way of swimming, the heart rate, or any data converted from the waveform). In the step 540, the output data are displayed.

In the step 550, the output data are stored for storing the output data.

In an embodiment, the output data may be transmitted to a receiving device (e.g., a computer device, a portable device, or a monitor that includes the microprocessor) that includes a game program through a network so that the output data is synchronized to a game program in the receiving device. In the step 560, the output data is transmitted to the receiving device through the network.

In the step 570, when the receiving device receives the output data through the network, the receiving device converts the output data received by the receiving module into the display data. In the step 580, the display data is displayed in the receiving device.

It will be apparent to those skilled in the art that various modifications and variations can be made to the structure of the present invention without departing from the scope or spirit of the invention. In view of the foregoing, it is intended that the present invention cover modifications and variations of this invention provided they fall within the scope of the following claims.

## Claims

**1.** A recognition device of a foot action, comprising:
a sensing module configured to sense the foot action to generate at least one sensing signal corresponding to the foot action which a form of the sensing signal is a waveform;
a processing module electrically connected the sensing module and configured to recognize an action mode corresponding to the foot action according to the waveform, and the action mode corresponding to the foot action according to the waveform converted into output data; and
a display module electrically connected the processing module and configured to display the output data.

**2.** The recognition device of claim 1, wherein the sensing module is one selected from a gyroscope, an accelerometer, an electron compass, a heart rate sensor, a humidity sensor, and a global positioning system (GPS) sensor.

**3.** The recognition device of claim 1, further comprising:
a storage module electrically connected the processing module and configured to store the output data.

**4.** The recognition device of claim 1, further comprising:
a transmission module electrically connected the processing module and configured to transmit the output data to a receiving device through a network, and the transmission module is one selected from a wide area network (WAN) module, a bluetooth module, a ANT+ module, and a local area network (LAN) module.

**4.** The recognition device of claim 4, wherein the receiving device comprising:
a receiving module configured to receive the output data transmitted by the transmission module through the network;
a microprocessor electrically connected the receiving module and configured to convert the output data received by the receiving module into display data; and
a display unit electrically connected the microprocessor and configured to display the display data.

**6.** The recognition device of claim 1, wherein the action mode is one selected from a working mode, a stair climbing mode, and a bicycle mode, a swimming mode, and a sleeping mode, and the stair climbing mode is one selected from a stair ascending mode and a stair descending mode, and the swimming mode is one selected from a freestyle mode and a breaststroke mode.

**7.** The recognition device of claim 6, wherein the processing module recognizes the action mode as one of the working mode, the stair climbing mode, and the bicycle mode according to the waveform when a cycle of the waveform comprises only a wave crest that the normal vector value of the wave crest exceeds a determine value, and the processing module recognizes the action mode as the swimming mode according to the waveform when a cycle of the waveform comprises more two wave crests that the normal vector value of the wave crest exceeds the determine value.

**8.** The recognition device of claim 7, wherein when the action mode is one of the working mode, the stair climbing mode, and the bicycle mode, the processing module recognizes the action mode as the bicycle mode if the cycle of the waveform comprises a similar sinusoidal wave.

**9.** The recognition device of claim 8, wherein the processing module recognizes the action mode as one of the working mode, the stair ascending mode of the stair climbing mode, and the stair descending mode of the stair climbing mode according to a grounding interval of the waveform, the processing module recognizes the action mode as the stair ascending mode when the maximum value of the grounding interval of the waveform is larger than the zero normal vector value, the processing module recognizes the action mode as the stair descending mode when the maximum value of the grounding interval of the waveform is smaller than the zero normal vector value, and the processing module recognizes the action mode as the walking mode when the maximum value of the grounding interval of the waveform is equal to the zero normal vector value.

**10.** The recognition device of claim 7, wherein when the action mode is the swimming mode, the processing module recognizes the action mode as the freestyle mode of the swimming mode if the cycle of the waveform comprises more one sinusoidal wave.

**11.** A recognition method of a foot action, comprising:
sensing the foot action to generate at least one sensing signal corresponding to the foot action which a form of the sensing signal is a waveform;
recognizing an action mode corresponding to the foot action according to the waveform, and converting the waveform into output data corresponding to the action mode; and
displaying the output data.

**12.** The recognition method of claim 11, further comprising:
storing the output data.

**13.** The recognition method of claim 11, further comprising:
transmitting the output data to a receiving device through a network.

**14.** The recognition method of claim 13, wherein when the receiving device through the network receives the output data, the receiving device converts the output data received into display data, and the display data is displayed in the receiving device.

**15.** The recognition method of claim 11, wherein the action mode is one selected from a working mode, a stair climbing mode, and a bicycle mode, a swimming mode, and a sleeping mode, and the stair climbing mode is one selected from a stair ascending mode and a stair descending mode, and the swimming mode is one selected from a freestyle mode and a breaststroke mode.

**16.** The recognition method of claim 15, further comprising:
recognizing the action mode as one of the working mode, the stair climbing mode, and the bicycle mode according to the waveform when a cycle of the waveform comprises only a wave crest that the normal vector value of the wave crest exceeds a determine value; and
recognizing the action mode as the swimming mode according to the waveform when a cycle of the waveform comprises more two wave crests that the normal vector value of the wave crest exceeds the determine value.

**17.** The recognition method of claim 16, further comprising:
recognizing the action mode as the bicycle mode if the cycle of the waveform comprises a similar sinusoidal wave when the action mode is one of the working mode, the stair climbing mode, and the bicycle mode.

**18.** The recognition method of claim 17, further comprising:
recognizing the action mode as one of the working mode, the stair ascending mode of the stair climbing mode, and the stair descending mode of the stair climbing mode according to a grounding interval of the waveform.

**19.** The recognition method of claim 18, further comprising:
recognizing the action mode as the stair ascending mode when the maximum value of the grounding interval of the waveform is larger than the zero normal vector value;
recognizing the action mode as the stair descending mode when the maximum value of the grounding interval of the waveform is smaller than the zero normal vector value; and
recognizing the action mode as the walking mode when the maximum value of the grounding interval of the waveform is equal to the zero normal vector value.

**20.** The recognition method of claim 11, further comprising:
recognizing the action mode as the freestyle mode of the swimming mode if the cycle of the waveform comprises more one sinusoidal wave.
